# EUROPEAN PATENT SPECIFICATION

(11) **EP 1 976 820 B1**
(45) Date of publication and mention of the grant of the patent: **29.09.2010**
(21) Application number: 06830802.2
(22) Date of filing: 22.12.2006
(51) Int. Cl.: C07C 31/38, C07C 69/653, C08F 220/24, D06M 15/277

(54) **FLUOROUS TELOMERIC COMPOUNDS AND POLYMERS CONTAINING SAME**
FLUORHALTIGE TELOMERE VERBINDUNGEN UND POLYMER, DAS DIESE ENTHÄLT
COMPOSÉS TÉLOMÈRES FLUORÉS ET POLYMÈRES LES CONTENANT

(30) Priority: 10.01.2006 DE 102006001218
(43) Date of publication of application: 08.10.2008
(73) Proprietor: Clariant Finance (BVI) Limited, Road Town, Tortola (VG)
(72) Inventor: GÖTZ, Hans, 84489 Burghausen (DE); KNAUP, Wolfgang, 84508 Burgkirchen (DE); PROBST, Anton, 84567 Erlbach (DE); AHOLLINGER, Christian, 84329 Wurmannsquick (DE)
(74) Representative: Jacobi, Carola
(86) International application number: PCT/EP2006/070147
(87) International publication number: WO 2007/080055

(56) References cited:
- EP-A- 1 754 768
- WO-A-91/01791
- WO-A-97/06127
- WO-A-03/062521
- WO-A-2004/067579
- DE-A1- 19 516 907
- JP-A- 5 018 371
- JP-A- 2002 080 547
- US-A- 3 083 224
- US-A- 3 645 989
- US-A- 3 818 074
- US-A- 3 838 104
- US-A- 4 339 518
- US-A- 4 997 873
- MCBEE ET AL.: JOURNAL OF THE AMERICAN CHEMICAL SOCIETY, vol. 72, 24 October 1949 (1949-10-24), pages 5071-5073, XP002427686
- J.D. PARK ET AL.: JOURNAL OF ORGANIC CHEMISTRY, vol. 23, 28 February 1958 (1958-02-28), pages 1166-1169, XP002427687
- SIVARAMAN ET AL.: JOURNAL OF ORGANIC CHEMISTRY, vol. 69, no. 25, 13 July 2004 (2004-07-13), pages 8751-8757, XP002427688
- Z. SZLAVIK ET AL: JOURNAL OF FLUORINE CHEMISTRY, vol. 98, 24 August 1998 (1998-08-24), pages 83-87, XP002427689
- N. O. BRACE: JOURNAL OF FLUORINE CHEMISTRY, vol. 20, 27 July 1981 (1981-07-27), pages 313-327, XP002427690
- N.O. BRACE: JOURNAL OF ORGANIC CHEMISTRY, vol. 27, 14 March 1962 (1962-03-14), pages 3033-3038, XP002427691

## Description

Fluorochemicals are often used as surfactants or wetting agents and are widely used for the surface treatment of substrates. They find frequent utility for the oil-, water-, and soil-repellent finishing of fibrous substrates such as for example carpets, textiles, leather, nonwovens and paper and of hard substrates such as for example wood, metal or concrete. The imbibition of hydrophilic and hydrophobic liquids is reduced with substrates thus treated, and the removal of existing soils is promoted.

Perfluoroalkyl iodides obtained via telomerization of telogens with fluorinated monomers such as tetrafluoroethene, for example, are an important starting point for the preparation of fluorocompounds.

To be used as a surface-modifying substance, perfluoroalkyl iodides are typically first converted with ethene into a perfluoroalkylethyl iodide. The perfluoroalkylethyl iodide can then be converted with suitable reagents into the corresponding perfluoroalkylethyl alcohol. From the perfluoroalkylethyl alcohols, then, the corresponding (meth)acrylate monomers of the formula I can be prepared.

R_{F}CH₂CH₂OCOCR=CH₂ (I)

The preparation of fluorous acrylates and methacrylates satisfying the formula I from various derivatives of acrylic acid and methacrylic acid respectively is well known and documented.

Copolymers prepared from these fluorous acrylates are particularly useful for modifying surfaces to be oil, water and soil repellent, for example for finishing textiles or for coating leather and paper.

The fluorous monomers of the formula II

R_{F}SO₂NR'(CH2)ₙOCOCR=CH₂ (II)

are known for similar applications.

The fluorinated alkylsulphonic acid fluoride used in their synthesis is obtained via electrochemical fluorination.

It has been determined for both monomer types (I and II) that the coating of surfaces with longer and ideally straight-chain perfluoroalkyl chains which consist of 8-10 fluorinated carbon atoms leads to particularly low surface energies.

Fluorous compounds having a linear perfluoroalkyl chain with 8 fluorinated carbon atoms, including the monomers described above, can degrade to form perfluorooctanecarboxylic acid and perfluorooctanesulphonic acid, respectively. These degradation products are considered not further degradable and therefore are persistent. Moreover, these compounds are suspected of accumulating in living organisms.

There have therefore been various proposals in recent years for preparing environmentally compatible perfluoroalkyl compounds.

WO 02/16306 describes branched fluorous (meth)acrylates having the formula III

R_{F}(R_{F}')CHOCOCR=CH₂ (III)

having a straight-chain or branched perfluoroalkyl group R_{F} of 5 or fewer carbon atoms and a branched perfluoroalkyl chain R_{F}' of 3 to 5 carbon atoms. These compounds lead specifically to degradation products of low molecular weight and low toxicity.

It is known that shorter-chain perfluoroalkylsulphonic acid derivatives are more easily eliminated from the body of living organisms. The WO 03/062521 patent describes textile finishes based on perfluorobutanesulphonic acid derivatives conforming to the formula II

R_{F}SO₂NR'(CH2)ₙOCOCR=CH₂ (II)

in lieu of perfluorooctanesulphonic acid derivatives having a partially branched perfluoroalkyl radical R_{F} of 4 fluorinated carbon atoms, n = 1, 2 and R' = H, alkyl and R = H, CH₃.

Compounds having a fluorinated alkyl radical of 4 carbon atoms and conforming to the formula I

R_{F}CH₂CH₂OCOCR=CH₂ (I)

are described in EP-1 632 542. It is likely that the degradation products are more easily eliminated from the body of living organisms.

WO 02/34848 describes the use of polyoxetanes having trifluoromethyl groups or pentafluoroethyl groups as perfluoroalkyl radical. This class of compounds likewise represents environmentally compatible perfluoroalkyl-containing compounds used as fluorosurfactants or for coatings.

WO 2004/060 964 describes fluorinated polyethers having a molecular weight of greater than 750 g/mol, which are eliminated particularly easily from the body of living organisms. WO 03/100 158 describes the use of such alcohols and acrylates for finishing textiles.

However, it has emerged that the heretofore described proposals for environmentally friendly alternatives to perfluoroalkyl compounds are less effective than them when used as a basis for oil- and water-repellent finishes. This is reflected in the values achieved for water repellency and oil repellency and in coating durability.

It is an object of the present invention to provide an alternative to polyfluoroalkyl-containing compounds and their derivatives which have no bioaccumulative effect. Its performance profile further includes a high effectivity when they are used for oil- and water repellent coatings. In addition, the compounds have to remain handlable on an industrial scale.

It has now been found that, surprisingly, polyfluoroalkyl compounds as hereinbelow defined lead to oil- and water-repellent coatings of high efficiency and durability and are also environmentally compatible.

The invention accordingly provides fluorous alcohol-, methacrylate- and/or acrylate-functionalized telomeric compounds having molecular weights of greater than 750 g/mol.

The invention further provides fluorous compounds which, owing to their being composed of a polyfluoroalkyl chain which is partly branched and partly linear, melt at lower temperatures than their molecular weight equivalents consisting of a linear polyfluoroalkyl chain.

The invention further provides fluorous alcohol-, methacrylate- or acrylate-functionalized telomeric compounds which are prepared from the corresponding fluorous alkyl iodides in one or more steps by telomerization reactions.

The invention further provides for the production of copolymers of the methacrylate- or acrylate-functionalized telomeric compounds.

The invention further provides for the use of the specified compounds or of their copolymers for applications in which the surface energy of a substrate is lowered.

The invention further provides for the use of the herein described copolymers for producing compositions used in the oil, water and soil repellency of fibrous substrates such as for example carpets, textiles, leather, nonwovens and paper and of hard substrates such as for example wood, metal or concrete.

The present invention provides fluorous telomeric compounds of the formula IV:

R_{F} - A - [CH₂]_{c}CR₂R₃ - Z (IV)

where R_{F} is a perfluoroalkyl radical of 1 to 20 carbon atoms,
A is a group of the formulae 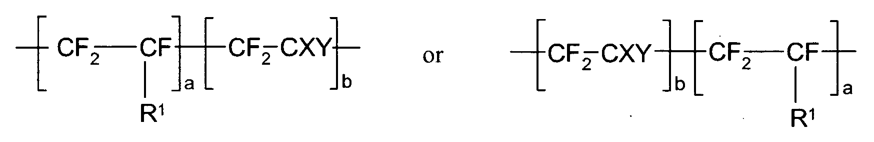 R¹ is CF₃ OR₄, Cl, Br or I,
R₂ and R₃ are H, alkyl or aryl
R₄ is perfluoromethyl, perfluoropropyl or perfluoropropyloxypropyl
X and Y are H, Cl or F
Z is -OH, -OCOCH=CH₂ or -OCOCCH₃=CH₂
a is from a>0 to 10, b is from 1 to 30 and c is from 1 to 30,
wherein the fluorous compounds of the formula IV have a molecular weight of greater than 750 g/mol. Particular preference is given to compounds of the formula IV which have a molecular weight of greater than 1000 g/mol.

The polyfluoroalkyl radical R_{F} can be a polyfluoroalkyl group having a unitary chain length or a mixture of polyfluoroalkyl groups having different chain lengths, for example CF₃, C₂F₅, C₃F₇, C₄F₉, C₆F₁₃, C₈F₁₇, C₁₀F₂₁, C₁₂F₂₅, C₁₄F₂₉ and C₁₆F₃₁ groups. The polyfluoroalkyl radical can be branched or unbranched.

Preference is given to compounds in accordance with the invention which have a saturated polyfluoroalkyl radical R_{F} which has a chain length of 1 to 20 fluorinated carbon atoms and comprises at least one terminal CH₃ group.

Particular preference is given to a fully fluorinated carbon chain R_{F} of 1 to 3 or 4 to 16 fluorinated carbon atoms.

R₁ is a sterically voluminous group which has a crystallization-inhibiting effect on the polyfluoroalkyl chain. Particular preference is given either to a perfluoromethyl group, to a perfluoroalkyl ether group or to a chlorine, bromine or iodine atom. A perfluoromethyl group is most preferable.

R₂ and R₃ can each be a hydrogen, an aryl group (phenyl) or a allyl chain of 1 to 4 carbon atoms. Preferably, R₂ and R₃ are each hydrogen or methyl.

More preferably, R₄ is a perfluoromethyl group, a perfluoropropyl group or a perfluoropropyloxypropyl group. A perfluoromethyl group is most preferable.

Preferably a is from a>0 to 5.

Preferably, b is from 1 to 30, more preferably a + b > 3.

Preferably, c is 1 to 30 and more preferably c = 1.

X and Y can independently be H, Cl or F. Preferably, X and Y are fluorine atoms. Alternatively X is a fluorine atom and Y a chlorine atom or X and Y are hydrogen atoms.

The fluorous alcohol of the formula IV (Z = OH) is typically obtained on the corresponding polyfluoroalkyl-alkyl iodide in a multistage process.

In the first step of the process, known as telomerization, a fluorous compound (telogen) capable of transferring a free radical chain is reacted with at least one fluorinated monomer (taxogen) via a free radical forming mechanism at 20-250°C to form the telomer of the formula

R_{F} - A - I.

Useful telogens include fluorous alkyl compounds having a group to be scissioned free-radically, for example fluorous alkyl iodides, bromides, thiols, thioethers and alcohols. Preference is given to perfluoroalkyl iodides having a unitary chain length or to a mixture of perfluoroalkyl iodides having different chain lengths. The perfluoroalkyl radical can be branched or unbranched, for example perfluoromethyl iodide, perfluoroethyl iodide, n-perfluoropropyl iodide, isoperfluoropropyl iodide, n-perfluorobutyl iodide, isoperfluorobutyl iodide, tert-perfluorobutyl iodide and isomers of perfluorohexyl iodide, perfluorooctyl iodide, perfluorodecyl iodide and perfluorododecyl iodide and so on.

Preference is given to perfluoroalkyl iodides in accordance with the invention having a chain length of 1 to 20 carbon atoms and at least one terminal CH₃ group.

Particular preference is given to perfluoromethyl iodide, perfluoroethyl iodide, perfluoropropyl iodide or perfluoroisopropyl iodide or a technical grade mixture of various perfluoroalkyl iodides, having chain lengths of 6 to 16 fluorinated carbon atoms or 8 to 16 fluorinated carbon atoms and an average chain length of about 7.5 fluorinated carbon atoms or about 9 fluorinated carbon atoms.

The addition of the taxogens onto the telogen results in the building up of higher molecular weights. The telomer thus formed consists of a perfluoroalkyl chain having a terminal iodine group. The way the taxogens are incorporated in the telomer differs according to which of the following three variants is chosen.

In the first variant, initially only a fluorinated unsaturated monomer CF₂=CFR₁ is added onto the telomer. The product then adds under the telomerization conditions the monomers of the formula CF₂=CXY. The telomer thus obtained has the formula 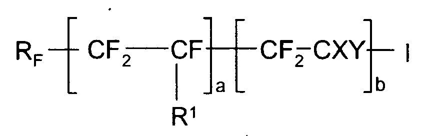 and exhibits blockwise incorporation of the monomers.

In the second variant, initially only a fluorinated unsaturated monomer CF₂=CXY is added. The product then adds under the telomerization conditions the monomers of the formula CF₂=CFR₁. The resulting telomer 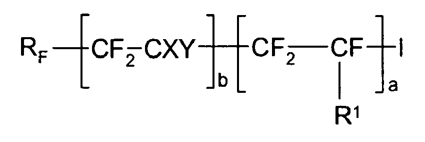 likewise exhibits blockwise incorporation of the monomers, but with the added monomers in the reverse order.

In the third variant, concurrent addition of a mixture of the two monomers results in random incorporation of the monomers CF₂=CFR₁ and CF₂=CXY.

Examples of compounds of the formula CF₂=CFR₁ are: chlorotrifluoroethene, bromotrifluoroethene, iodotrifluoroethene, perfluoromethyl vinyl ether, perfluoroethyl vinyl ether, perfluoropropyl vinyl ether, perfluoropropyloxypropyl vinyl ether and also branched and unbranched perfluoroolefins having a terminal double bond, examples being hexafluoropropene, 1-perfluorobutene, 1-perfluorohexene or perfluorooctene.

Examples of compounds of the formula CF₂=CXY are for example-tetrafluoroethene, vinylidene fluoride, chlorotrifluoroethene, trifluoroethene, 1,1-dichloro-2,2-difluoroethene and 1-chloro-2,2-difluoroethene.

In the case of iodine-containing compounds, free radicals which initiate the telomerization reactions can be generated by sources capable of forming free radicals. Useful sources for forming free radicals include light or heat. The light source typically has its maximum in the infrared to ultraviolet region. Free radical formation due to heat typically takes place at temperatures between 100°C and 250°C.

Useful sources for forming free radicals further include free radical initiators of the chemical kind, which are also capable of lowering the reaction temperature required for free radical formation to between 0°C and 150°C, examples being organic or inorganic peroxides, azo compounds, organic and inorganic metal compounds and metals and also combinations thereof. Particular preference is given to persulphates, fluorinated and nonfluorinated organic peroxides, azo compounds and metals such as for example Ru, Cu, Ni, Pd and Pt.

The telomerization can be carried solventlessly, in solution, in suspension or emulsion. The reaction without a solvent or in emulsion is particularly preferred. In the case of the reaction in emulsion, the telogen is first converted with the aid of surfactants into an aqueous emulsion. The emulsion can be stabilized by anionic, cationic, nonionic or amphoteric surfactants and combinations thereof. Fluorosurfactants are particularly suitable for example. The reaction typically takes place at elevated temperature through addition of the taxogens and free radical initiators. Additional components can increase the reaction yield, examples being small amounts of aqueous solutions of sulphites, bisulphites or dithionates.

In the second step of the process, the polyfluoroalkyl iodide thus obtained is reacted with an olefin under free radical conditions to obtain the corresponding substituted or unsubstituted polyfluoroalkylethyl iodide of the formula

R_{F} - A - CH₂CR₂R₃I

The insertion of these olefins proceeds via a free radical process, which can be carried out similarly to the telomerization reaction described above.

Preferred olefins for the addition reaction can be ethylenically unsaturated compounds, for example ethene, propene, 1-butene, isobutene, 1-hexene, 1-octene, 1-decene, 1-undecene, 1-dodecene, 1-tetradecene, 1-hexadecene, 1-octadecene, styrene and 1,1-diphenylethene. Ethene is particularly preferred.

In the third step of the process, the substituted or unsubstituted polyfluorinated ethyl iodide is converted into the corresponding polyfluoroalkylethyl alcohol of the formula

R_{F} - A - CH₂CR₂R₃OH

For this, the polyfluoroalkylethyl iodide is hydrolysed with suitable coreactants to form the alcohol, as for example with the aid of p-toluenesulphonic acid, nitric acid, sulphuric acid, N-methylpyrrolidone, acid amides and peroxy acids. In a particularly preferred embodiment of the hydrolysis to form the alcohol, the polyfluoroalkylethyl iodide is reacted with N-methylpyrrolidone and water at temperatures between 100°C and 200°C.

In another aspect of the invention, the polyfluoroalkyl iodide of the first step is used to prepare the corresponding substituted or unsubstituted polyfluoroalkylalkyl alcohol of the formula

R_{F} - A - [CH₂]_{c}CR₂R₃OH

In effect, the polyfluoroalkyl iodide is added onto γ-alkenols in the manner described above, under the appropriate conditions. The iodine-containing polyfluoroalcohols thus obtained can subsequently be converted by a prior art hydrogenation into a saturated polyfluoroalkylalkyl alcohol. Examples of the γ-alkenols used are γ-propenol, γ-butenol, γ-pentenol, γ-hexenol, γ-heptenol, γ-decenol and γ-undecenol.

Alternatively, the polyfluoroalkylethyl iodide of the second step is used to prepare the corresponding substituted or unsubstituted polyfluoroalkylpropyl alcohol of the formula

R_{F}- A - [CH₂]₂CR₂R₃OH.

For this, the polyfluoroalkylethyl iodide is converted into the corresponding polyfluoroalkylolefin by dehydroiodation and subsequently reacted, with the aid of a free radical initiator, with an aliphatic alcohol such as, for example, methanol, ethanol, butanol or isopropanol to form a correspondingly substituted or unsubstituted polyfluoroalkylpropyl alcohol.

The polyfluoroalkylalkyl alcohols obtained in these various ways can be reacted with (meth)acrylate esters, acids or acid chlorides to form the corresponding fluorous (meth)acrylates of the formula

R_{F} - A - [CH₂]_{c}CR₂R₃OCOCH=CH₂ or R_{F} - A - [CH₂]_{c}CR₂R₃OCOCCH₃=CH₂.

The reaction with the (meth)acrylate acid chlorides is typically carried out in the presence of a base such as triethylamine to bind hydrogen chloride formed. A suitable catalyst, for example a tin catalyst, can be used for the transesterification.

These (meth)acrylates can be copolymerized with nonfluorous polymerizable vinyl monomers, thermally crosslinkable or isocyanate-reactive monomers and chlorine-containing polymerizable vinyl monomers.

The invention also provides copolymers containing, based on the total weight of the copolymer:
a) 20% to 97% by weight and preferably 40% to 90% by weight of a monomer of the formula IV where Z is -OCOCH=CH or -OCOCCH₃=CH,
b) 0% to 80% by weight and preferably 10% to 50% by weight of one or more nonfluorous polymerizable vinyl monomers and/or
c) 0.5% to 20% by weight and preferably 1% to 10% by weight of one or more thermally crosslinkable or isocyanate-reactive monomers.

The present invention further provides copolymers containing, based on the total weight of the copolymer:
a) 40% to 90% by weight and preferably 45% to 85% by weight of a monomer of the formula IV where Z is -OCOCH=CH or -OCOCCH₃=CH,
b) 0% to 50% by weight and preferably 0.01% to 30% by weight of one or more nonfluorous polymerizable vinyl monomers and/or
c) 0.5% to 20% by weight and preferably 1 % to 10% by weight of one or more thermally crosslinkable or isocyanate-reactive monomers and
d) 0.5% to 50% by weight and preferably 2% to 30% by weight of a chlorine-containing polymerizable vinyl monomer.

The optional comonomer (b) is not fluorous (does not contain fluorine) and can be represented by a multiplicity of commercially available acrylates and methacrylates and styrene derivatives.

Examples of nonfluorinated comonomers are hydrocarbyl esters and amides of unsaturated carboxylic acids. These include for example the following esters and amides of acrylic acid, methacrylic acid, α-chloroacrylic acid, crotonic acid, maleic acid, fumaric acid and itaconic acid: vinyl, allyl, methyl, ethyl, propyl, isopropyl, n-butyl, isobutyl, t-butyl, hexyl, 3,3-dimethylbutyl, heptyl, octyl, isooctyl, lauryl, cetyl, stearyl, behenyl, cyclohexyl, bornyl, isobornyl, phenyl, benzyl, adamantyl, tolyl, (2,2-dimethyl-1-methyl)propyl, cyclopentyl, 2-ethylhexyl, 4-ethylcyclohexyl, 2-ethoxyethyl and tetrahydropyranyl.

Further nonfluorinated comonomers are allyl esters and vinyl esters such as for example allyl acetate, vinyl acetate, allyl heptanoate and vinyl heptanoate; alkyl vinyl ethers and alkyl allyl ethers such as for example cetyl vinyl ether, dodecyl vinyl ether, octadecyl vinyl ether and ethyl vinyl ether; α,β-unsaturated nitriles such as for example acrylonitrile, methacrylonitrile, α-chloroacrylonitrile, α-cyanoethyl acrylate; aminoalkyl (meth)acrylates such as for example N,N-diethylaminoethyl (meth)acrylate, N-t-butylaminoethyl (meth)acrylate; alkyl (meth)acrylates having an ammonium group such as for example 2-methacryloyloxyethyltrimethylammonium chloride; styrene and its derivative such as for example vinyltoluene, α-methylstyrene, α-cyanomethylstyrene, chloromethylstyrene; olefinic hydrocarbons such as for example ethene, propene, isobutene, butadiene, isoprene; and (meth)acrylates of methoxy polyethylene glycols.

Particularly preferred optional comonomers (b) can be the following esters or amides of acrylic acid and methacrylic acid: methyl, ethyl, propyl, butyl, isobutyl, 2-ethylhexyl, myristyl, lauryl, octadecyl, methoxy poly(ethylene glycol) and methoxy poly(propylene glycol) as described above.

The comonomer (c) contains one or more crosslinkable groups. A crosslinkable group is a functional group capable of entering a reaction with the substrate and/or with a further polyfunctional compound added. Such crosslinkable groups can be: carboxylic acid groups, ethylenically unsaturated groups, hydroxyl groups, amino groups, N-alkylolamide groups, isocyanate groups or protected isocyanate groups. Examples of comonomers having one or more crosslinkable groups include unsaturated carboxylic acids and anhydrides of acrylic acid, methacrylic acid, α-chloroacrylic acid, crotonic acid, maleic acid, fumaric acid and itaconic acid, monomers including a hydroxyl group, for example hydroxyethyl (meth)acrylates and hydroxypropyl (meth)acrylates, hydroxybutyl (meth)acrylate, poly(ethylene glycol) mono(meth)acrylate, poly(propylene glycol) mono(meth)acrylate, poly(ethylene glycol)-co-poly(propylene glycol) mono(meth)acrylate, polytetrahydrofuran mono(meth)acrylate, N-hydroxymethyl(meth)acrylamide, hydroxybutyl vinyl ether. Further crosslinkable monomers are for example vinyl (meth)acrylate, allyl (meth)acrylate, N-methoxymethylacrylamide, N-isopropoxymethylacrylamide, N-butoxymethylacrylamide, N-isobutoxymethylacrylamide, glycidyl (meth)acrylate and α,α-dimethyl-m-isopropenylbenzyl isocyanate. Other examples are monomers which release isocyanates at elevated temperatures or under irradiation with light, examples being phenol-, ketoxime- and pyrazole-protected isocyanate-terminated alkyl (meth)acrylates.

The optional comonomer (d) is chlorine containing. Examples of chlorine-containing comonomers are halogenated olefinic hydrocarbons such as for example vinyl chloride, vinylidene chloride, 3-chloro-1-isobutene, 1-chlorobutadiene, 1,1-dichlorobutadiene and 2,5-dimethyl-1,5-hexadiene. Vinylidene chloride and vinyl chloride are particularly preferred optional comonomers (c).

The copolymer described hereby is typically prepared by a free radical polymerization technique, for example by solvent, emulsion, microemulsion or miniemulsion polymerization techniques. Variants of the emulsion polymerization are particularly preferred. The emulsion polymerization of the monomers takes place in the presence of water, surfactants and an optional organic solvent. The mixture can have been pre-emulsified before the polymerization, by means of a high pressure homogenizer or a similar apparatus. The polymerization is typically carried out at temperatures between 50°C and 150°C in the presence of a free radical initiator.

Various anionic, cationic, nonionic or amphoteric surfactants can be employed, alone or in combination. Examples of nonionic surfactants include poly(ethylene glycol) lauryl ether, poly(ethylene glycol) tridecyl ether, poly(ethylene glycol) cetyl ether, poly(ethylene glycol)-co-poly(propylene glycol) cetyl ether, poly(ethylene glycol) stearyl ether, poly(ethylene glycol) oleyl ether, poly(ethylene glycol) nonylphenol ether, poly(ethylene glycol) octylphenol ether, poly(ethylene glycol) monolaurate, poly(ethylene glycol) monostearate, poly(ethylene glycol) monooleate, sorbitan monolaurate, sorbitan monostearate, sorbitan monopalmitate, sorbitan monostearate, sorbitan monooleate, sorbitan sesquioleate, sorbitan trioleate, poly(ethylene glycol) sorbitan monolaurate, poly(ethylene glycol) sorbitan monopalmitate, poly(ethylene glycol) sorbitan monostearate, poly(ethylene glycol) sorbitan monooleate, poly(ethylene glycol)-co- poly(propylene glycol), polyglycerol fatty acid esters, polyether-modified silicone oils and perfluoroalkyl-ethylene oxide adducts. The amount of nonionic surfactant used ranges from 0.1 to 100 percent by weight, relative to the weight of the polymer.

Examples of the cationic surfactants in accordance with the invention are ammonium compounds based on saturated and unsaturated fatty acid amines, for example octadecylammonium acetate, dodecyltrimethylammonium chloride; ammonium compounds based on amino-functionalized polyethoxylates and polypropoxylates and their interpolymers such as for example polyoxyethylene laurylmonomethylammonium chloride; ammonium compounds based on arylamines such as for example biphenyltrimethylammonium chloride, imidazoline derivatives such as for example ammonium salts formed from tallow and imidazoline; silicone-based cationic surfactants and fluorine-based cationic surfactants. The amount of cationic surfactant used ranges from 0.1 to 100 percent by weight relative to the weight of the polymer.

Examples of the anionic surfactants in accordance with the invention include fatty alcohol sulphates, for example sodium dodecylsulphate and poly(ethylene glycol) lauryl ether sulphate; alkylsulphonates such as for example sodium laurylsulphonate; alkylbenzenesulphonates, for example nonylphenol ether sulphates, sulphosuccinates, for example sodium hexyl diether sulphosuccinate; fatty alcohol phosphates, for example sodium laurylphosphate and fatty acid salts, such as for example sodium stearic acid salt. The amount of anionic surfactant used ranges from 0.1 to 100 percent by weight, relative to the weight of the polymer.

Examples of free radical initiators are organic or inorganic peroxides, azo compounds, organic and inorganic metal compounds and metals and also combinations thereof. Particular preference is given to azo compounds such as azobisisobutyronitriles (AIBNs), azobisvaleronitrile and azobis(2-cyanovaleric acid), 2,2'-azobis(2-amidinopropane) dihydrochloride; hydroperoxides such as cumene hydroperoxide, t-butyl hydroperoxide and t-amyl hydroperoxide, dialkyl peroxides such as di-t-butyl peroxide and dicumyl peroxide, peroxyesters such as t-butyl perbenzoate and di-t-butyl peroxyphthalate, diacyl peroxides, such as benzoyl peroxide and lauroyl peroxide; inorganic peroxides such as ammonium persulphate and potassium persulphate and also combinations of the specified compounds with organic or inorganic metal compounds and metals.

A chain transfer agent can be used in the polymerization, an example being an alkylthiol.

Examples of the organic solvent in the solvent and emulsion polymerization are: ketones such as for example acetone, methyl ethyl ketone and methyl isobutyl ketone; alcohols such as for example ethanol, isopropanol and butanol, polyalcohols such as for example 1,3-butanediol, 1,6-hexanediol, ethylene glycol, propylene glycol, dipropylene glycol, tripropylene glycol and glycerol; ethers and esters of polyalcohols, such as for example dipropylene glycol monomethyl ether, tripropylene glycol monomethyl ether, triethylene glycol dimethyl ether and diethylene glycol monobutyl ether acetate; esters such as for example ethyl acetate, propyl acetate, butyl acetate, dibutyl adipate and dibutyl succinate; hydrocarbons and halogenated hydrocarbons such as for example toluene, xylene, octane, perchloroethylene and 1,3-dichloro-2,2,3,3,3-pentafluoropropane.

The preferred solids content for the polymer dispersion prepared is between 20 and 40 percent by weight.

The fluorous copolymers containing a fluorous monomer of the formula IV are suitable for coating fibrous substrates such as for example carpets, textiles, leather, nonwovens or paper or hard substrates such as for example wood, metal or concrete. They endow these substrates with water-, oil- and soil-repellent properties.

The invention thus also provides a process for surface treatment of fibrous substrates with an effective amount of the fluorous aqueous dispersion.

The content of the preparation for finishing textiles and other sheetlike structures in accordance with this invention is chosen so that sufficient repellent properties are transferred to the treated substrate. The wet pick-up was determined by weighing the finished specimens before and after application.

The fluorous textile-finishing agents according to the invention can be used together with other additives, including water-repellent materials, such as for example waxes, silicones, zirconium compounds or stearic acid salts, and also other oil-repellent materials, surfactants, insecticides, flame retardants, antistatic additives, plasticizers, dye fixatives and crease resist additives in an amount which does not impair fixing on the textile and the stability of the composition.

The fluorous textile-finishing agents according to the invention can be crosslinked by addition of reactive additives such as for example melamine resins, protected isocyanates or epoxides.

The fibrous substrates to be coated with the fluorous polymeric dispersion can be for example carpets, textiles, leather, nonwovens and paper. These consist inter alia of natural fibres such as for example cotton, linen and silk; of synthesis fibres such as for example polyamides, polyesters, polyurethanes, polyolefins, poly(meth)acrylates, poly(vinyl chlorides), poly(vinyl alcohols); semisynthetic fibres such as for example rayon or acetate; inorganic fibres such as for example glass fibres or ceramic fibres or any desired combination of the specified fibres or any desired combination of woven products composed of these materials.

For coating, the substrate is typically immersed in a dilute dispersion consisting of copolymer and optional additives. Alternatively, the dilute dispersion can be sprayed onto the substrate. The saturated substrate is subsequently pressed by a system of rolls to remove excess dispersion, dried in an oven and crosslinked at a temperature and for a time sufficient to ensure crosslinking on the treated substrate. This crosslinking process is typically carried out at temperatures between 50 and about 190°C. In general, a temperature of about 120°C to 180°C and in particular of about 130°C to 170°C for a period of 20 seconds up to 10 minutes is suitable, preference being given to 5 seconds to 5 minutes.

A further alternative for applying the preparation to a substrate is foam application wherein the preparation is applied to the substrate as a foam which is then dried and crosslinked. For foam application, the preparation is typically added in a concentrated form which has been admixed with an additional foamer. A highly concentrated preparation for foam application typically contains the fluoropolymer in an amount of up to 20% by weight.

For the finishing on textiles, these can be examined in specific tests for their water-, isopropanol- and oil-repellent properties before and after washing.

Water repellency is attained by the spray test as per AATCC Standard Test Method 22. Distilled water is sprayed onto the textile substrate to be tested and a subsequent visual comparison of the pattern of wetting with reference pictures of an evaluation standard recited in the test method was used to generate a numerical value. The reported numerical values relate to the appearance of the surface after spraying with water and have the following connotation (Table 1):

**TABLE 1**

| Water repellency rating | Connotation |
|---|---|
| 100 | No clinging of water droplets or wetting of the upper surface |
| 90 | Occasional clinging of water droplets or wetting of the upper surface |
| 80 | Wetting of the upper surface at water impact points |
| 70 | Partial wetting of total upper surface |
| 50 | Complete wetting of total upper surface |
| 0 | Complete wetting of total upper and lower surfaces |

A second test with a series of water-isopropanol test solutions can be used to determine the isopropanol repellency (IPA) of a substrate. The reported IPA rating is the highest numbered test solution where the fabric is not wetted within 10 seconds and the drops still have the shape of a sphere or a hemisphere. Wetted substrates or substrates which are only repellent to 100% water (0% isopropanol), i.e. the least wetting test solution, are rated 0, whereas substrates which are repellent to 100% isopropanol (0% water) are rated 10. Intermediate ratings can be assigned as well.

Oil repellency as per AATCC Standard Test Method 118 tests the ability of a substrate to repel oily soiling, higher ratings in the assessment scale denoting better repellency of such soil, in particular of oily liquids. In the test, drops of standardized test liquids, consisting of a selected series of hydrocarbons having different surface tensions, are applied in succession to the surface of the specimen to be tested, by careful pipetting, and the wetting is visually assessed after a defined contact time. The oil repellency value corresponds to the highest numbered test liquid which causes no wetting of the surface. The standard test liquids have the following composition (Table 2):

**TABLE 2**

| Oil repellency | Composition |
|---|---|
| Rating 1 | Nujol^{®} |
| Rating 2 | 65 vol% of Nujol/35 vol% of n-hexadecane |
| Rating 3 | n-hexadecane |
| Rating 4 | n-tetradecane |
| Rating 5 | n-dodecane |
| Rating 6 | n-decane |
| Rating 7 | n-octane |
| Rating 8 | n-heptane |

| | |
|---|---|
| Note: Nujol is a mineral oil from Plough Inc. having a Saybolt viscosity of 360/390 at 38°C and a specific weight of 0.880/0.900 at 15°C. | |

Prior art FC polymers are currently giving oil repellency values of 6; however, a rating of 5 is usually already considered excellent.

### EXAMPLES

The examples which follow illustrate the subject matter and advantages of the invention, but the materials and amounts cited in the examples shall not be viewed as limiting.

### SYNTHESES

### EXAMPLE 1:

### Synthesis of C₈F₁₇(CF₂CF(CF₃))ₐ(CF₂CF₂)_{b}I

An emulsion of 110 g (0.18 mol) of Fluowet 1812* (Clariant), 15 g of Fluorolink C (Solvay Solexis), 5 g of ammonia and 90 g of water was prepared by intensive stirring at 60°C and introduced into an autoclave as an initial charge together with 2.5 g of ammonium persulphate. The pressure test was followed by repeated purging with nitrogen. During the heating-up phase to 80°C, hexafluoropropene and tetrafluoroethene were added to the stirred emulsion in a ratio of 3:5 up to an overall pressure of 17 bar. The pressure is kept constant at 17 bar until 82.5 g (0.55 mol) of hexafluoropropene and 90 g (0.90 mol) of tetrafluoroethene have been added. After a drop in pressure, the autoclave is cooled down to room temperature and the fluorochemical phase is separated off by addition of salt and washed. The low molecular weight constituents are separated off by distillation. The iodine content of 11.2% suggests an average molecular weight of about 1400 g/mol.

¹⁹F NMR (solvent CDCl₃/C₆F₆, versus CFCl₃): -59.8 (2F, -CF₂I), -71.8 to -77.0 (in each case 3F, -CF-CF₃), -81.9 (3F, -CF₂-CF₃), -110.2 to -126.9 (in each case 2F, -CF₂-), -184.6 to -185.5 (in each case 1F, -CF(CF₃)-).

It is evident from the ¹⁹F NMR spectrum that about 2 molecules of hexafluoropropene have been incorporated per perfluoroalkyl iodide used.

* The compound designated Fluowet I812 is a perfluoroalkyl iodide mixture having 6 to 14 fluorinated carbon atoms per molecule having an average chain length of about 9 fluorinated carbon atoms.

Fluorolink C is a perfluoro polyether carboxylic acid.

### EXAMPLES 2 TO 10:

### Synthesis of polyfluoroalkyl iodides

Example 1 was repeated to prepare corresponding polyfluoroalkyl iodides (Examples 2 to 10). The results of the syntheses are shown in Table 3.

**TABLE 3**

| | | | | | | | |
|---|---|---|---|---|---|---|---|
| Telomerization reactions to prepare polyfluoroalkyl iodides having the general composition: | | | | | | | |
| 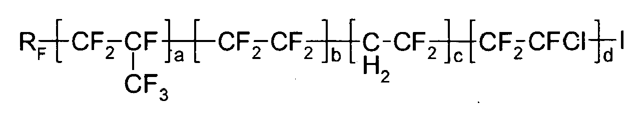 | | | | | | | |

| Ex. No. Iodide | R_{F} | R_{F} [mol] | a [mol] | b [mol] | c [mol] | d [mol] | Mₙ * [kg/mol] |
|---|---|---|---|---|---|---|---|
| 2 | (CF₃)₂CF- | 0.25 | 0.55 | 2.23 | - | - | 1.3 |
| 3 | C₈F₁₇- | 0.25 | 0.71 | - | 3.06 | - | 1.5 |
| 4 | C₂F₅- | 0.20 | 0.43 | 4.04 | - | - | 2.4 |
| 5 | C₈F₁₇- | 0.35 | 0.68 | - | - | 1.75 | 1.2 |
| 6 | I812- ** | 0.30 | - | 2.49 | - | 1.23 | 1.7 |
| 7 | C₈F₁₇- | 0.25 | - | - | 1.98 | 1.01 | 1.3 |
| 8 | (CF₃)₂CF- | 0.18 | 0.20 | - | 1.83 | - | 0.9 |
| 9 | I612- ** | 0.22 | 0.87 | 1.32 | - | - | 1.5 |
| 10 | C₈F₁₇- | 0.26 | - | - | - | 2.07 | 1.8 |

| | | | | | | | |
|---|---|---|---|---|---|---|---|
| * determined from iodine content ** the compounds designated Fluowet I612 and Fluowet I812 are perfluoroalkyl iodide mixtures from Clariant, each having 6 to 14 fluorinated carbon atoms per molecule having an average chain length of about 7.5 fluorinated carbon atoms and 9 fluorinated carbon atoms respectively. | | | | | | | |

### EXAMPLE 11:

### Synthesis of C₈F₁₇(CF₂CF(CF₃))ₐ(CF₂CF₂)_{b}CH₂CH₂I

5 g of H 101 B/W ruthenium-containing catalyst (Degussa) and 173 g of the polyfluoroalkyl iodide of Example 1 were introduced as an initial charge under nitrogen. After a pressure test with 50 bar nitrogen, the autoclave was evacuated down to 1 mbar and then cooled down to -78°C. 10 g of ethene were then condensed in at - 78°C. The reaction mixture was shaken at 170°C for 30 hours. Decompression and filtration left 160 g of crude product.

¹H NMR (solvent CDCl₃/C₆F₆): 2.7 (2H, -CH₂CH₂I), 3.2 (2H, -CH₂CH₂I).

### EXAMPLES 12 TO 20:

### Synthesis of polyfluoroalkylalkyl iodides

Example 11 was repeated to prepare corresponding polyfluoroalkylalkyl iodides (Examples 12 to 20). The results of the syntheses are shown in Table 4.

**TABLE 4**

| | | | | | | | |
|---|---|---|---|---|---|---|---|
| Addition of olefins to prepare polyfluoroalkylalkyl iodides having the general composition (c = 1 for each): | | | | | | | |
| R_{F} - A - [CH₂]_{c}CR₂R₃I | | | | | | | |

| Ex. No. Ethyl iodide | Ex. No. Iodide | Olefin | Iodide [mol] | Olefin [mol] | Initiator [g] | R₂ | R₃ |
|---|---|---|---|---|---|---|---|
| 12 | 2 | ethene | 0.08 | 0.20 | 5.0 | -H | -H |
| 13 | 3 | propene | 0.10 | 0.20 | 5.0 | -H | -CH₃ |
| 14 | 4 | ethene | 0.08 | 0.20 | 5.0 | -H | -H |
| 15 | 5 | 1-butene | 0.10 | 0.30 | 5.0 | -H | -CH₂CH₃ |
| 16 | 6 | ethene | 0.05 | 0.10 | 2.5 | -H | -H |
| 17 | 7 | isobutene | 0.05 | 0.40 | 2.5 | -CH₃ | -CH₃ |
| 18 | 8 | ethene | 0.10 | 0.30 | 5.0 | -H | -H |
| 19 | 9 | ethene | 0.05 | 0.10 | 2.5 | -H | -H |
| 20 | 10 | ethene | 0.05 | 0.10 | 2.5 | -H | -H |

### EXAMPLE 21:

### Synthesis of C₈F₁₇(CF₂CF(CF₃))ₐ(CF₂CF₂)_{b}CH₂CH₂OH (method A)

65 g (0.07 mol) of the polyfluoroalkylethyl iodide of Example 11 were introduced as an initial charge under nitrogen together with 83 g of N-methylpyrrolidone and 25 g of demineralized water. Following a pressure test with 50 bar of nitrogen, the autoclave was shaken at 160°C for 24 hours. After decompression, the crude product was admixed with 0.5 1 of water. The fluorous phase was repeatedly washed with sodium chloride solution and dried to obtain the polyfluoroalkyl ethanol.

¹H NMR (solvent CDCl₃/C₆F₆): 2.4 (2H, -CH₂CH₂OH), 3.9 (2H, -CH₂CH₂OH).

### EXAMPLE 22:

### Synthesis of C₈F₁₇(CF₂CF(CF₃))ₐ(CF₂CF₂)_{b}CH₂CH₂CH₂OH (method B)

To prepare the polyfluoroalkylethene, 310 g (0.34 mol) of the polyfluoroalkylethyl iodide of Example 11 were gradually added dropwise to a cooled solution of 17.4 g of potassium hydroxide in 250 ml of ethanol at 0°C. The reaction mixture was stirred at room temperature for one hour. The potassium iodide formed was filtered off and the polyfluoroalkylethene was repeatedly washed with water and dried.

For the reaction to form the alcohol, 800.0 g (25.0 mol) of methanol were introduced as an initial charge in a stirrer-equipped autoclave, purged with nitrogen and heated to 160 DEG C. Then, 290.0 g of the polyfluoroalkylethene and 4.8 g of di-tert-butyl peroxide were placed in a feed vessel and continuously fed from the feed vessel to the heated methanol by means of a metering pump. The feed rate setting was such that the polyfluoroalkylethylene-peroxide mixture was added after 3 hours. The pressure in the autoclave was 21 bar. Following the metered addition, the mixture was held at the stated temperature for a further 2 hours of supplementary reaction. To recover the polyfluoroalkylpropanol product formed from the reaction mixture, the excess ethanol was distilled off and the bottom product, which constitutes the polyfluoroalkyl alcohol, was repeatedly washed with water.

¹H NMR (solvent CDCl₃/C₆F₆): 1.9 (2H, -CF₂CH₂CH₂-), 2.1 (2H, -CF₂CH₂CH₂-), 3.7 (2H, -CH₂CH₂OH).

### EXAMPLE 23:

### Synthesis of C₈F₁₇(CF₂CF(CF₃))ₐ(CF₂CF₂)_{b}(CH₂)₁₁OH (method C)

A solution of 65.0 g (0.05 mol) of the polyfluoroalkyl iodide of Example 1 and 10.0 g of 10-undecen-1-ol were heated to 80°C under nitrogen in a three neck flask equipped with reflux condenser. Then, 0.15 g of the initiator 2,2'-azo-bis-isobutyronitrile (AIBN) was added. The reaction was maintained at this temperature for one hour, at which point the same amount of AIBN was added. The reaction mixture was stirred at 80°C for a further 7 hours. The yield was 85%. After work-up by distillation, 20.0 g of the iodine-containing alcohol obtained was cooled down to 0°C, and purged with nitrogen, in a three neck flask. Then, 12.0 g of tributylstannane dissolved in 70 ml of tetrahydrofuran was slowly added dropwise. On completion of the addition, the mixture was warmed to room temperature and stirred for a further 3 hours. The product was obtained by washing out (yield 65%).

¹H NMR (solvent CDCl₃/C₆F₆): 1.2 to 1.8 (18H, -CF₂CH₂(CH₂)₉-), 2.2 (2H, - CF₂CH₂CH₂-), 3.8 (2H, -CH₂CH₂OH).

### EXAMPLES 24 TO 30:

### Synthesis of polyfluoroalkylalkyl alcohols

Examples 21, 22 and 23 (methods A, B, C) were repeated to prepare corresponding polyfluoroalkylalkyl alcohols (Examples 24 to 30). The results of the syntheses are shown in Table 5.

**TABLE 5**

| | | | | | | |
|---|---|---|---|---|---|---|
| Reactions to prepare polyfluoroalkylalkyl alcohols having the general composition: | | | | | | |
| R_{F} - A - [CH₂]_{c}CR₂R₃OH | | | | | | |

| Ex. No. Alkyl alcohol | Ex. No. Alkyl iodide or iodide | Method | Alkyl iodide or iodide [mol] | c | R₂ | R₃ |
|---|---|---|---|---|---|---|
| 24 | 12 | A | 0.05 | 1 | -H | -H |
| 25 | 13 | A | 0.05 | 1 | -H | -CH₃ |
| 26 | 14 | B | 0.05 | 2 | -H | -H |
| 27 | 15 | B* | 0.05 | 2 | -H | -CH₃ |
| 28 | 16 | A | 0.05 | 1 | -H | -H |
| 29 | 19 | A | 0.05 | 1 | -H | -H |
| 30 | 10 | C** | 0.05 | 3 | -H | -H |

| | | | | | | |
|---|---|---|---|---|---|---|
| * by reaction with ethanol ** by reaction with 1-butenol | | | | | | |

### EXAMPLE 31:

### Synthesis of C₈F₁₇(CF₂CF(CF₃))ₐ(CF₂CF₂)_{b}CH₂CH₂OCOCH=CH₂ (method D)

A three neck flask was charged with 93 g (0.06 mol) of the alcohol of Example 21, 25.0 g of acrylic acid, 0.3 g of methanesulphonic acid and 0.4 g of p-methoxyphenol and this initial charge was heated to 80°C. The water of reaction was separated from the reaction within 24 hours at the reaction temperature and a pressure of 200 mbar. The organic phase was repeatedly washed with warm water and dried in a rotary evaporator.

¹H NMR (solvent CDCl₃/C₆F₆): 2.1 (2H, -CH₂CH₂OH), 4.4 (2H, -CH₂CH₂O-), 5.8 to 6.5 (3H, -CH=CH₂).

### EXAMPLE 32:

### Synthesis of C₈F₁₇(CF₂CF(CF₃))ₐ(CF₂CF₂)_{b}CH₂CH₂OCOC(CH₃)=CH₂ (method E)

A three neck flask was charged with 80 g (0.06 mol) of the alcohol of Example 21, 25 g of methacrylic acid, 0.3 g of methanesulphonic acid and 0.4 g of p-methoxyphenol and this initial charge was heated to 80°C. The water of reaction was separated from the reaction within 24 hours at the reaction temperature and a pressure of 200 mbar. The organic phase was repeatedly washed with warm water and dried in a rotary evaporator.

¹H NMR (solvent CDCl₃/C₆F₆): 1.9 (3H, -CH₃), 2.5 (2H, -CH₂CH₂O-), 4.4 (2H, -CH₂CH₂O-), 5.6 to 6.2 (2H, -C(CH)₃=CH₂).

### EXAMPLES 33 TO 40:

### Synthesis of polyfluoroalkyl acrylates

Examples 31 and 32 were repeated to convert the polyfluoroalkyl alcohols into polyfluoroalkyl (meth)acrylates (Examples 33 to 40). The composition is discernible from Table 6.

**TABLE 6**

| | | | | | |
|---|---|---|---|---|---|
| Reactions to prepare polyfluoroalkyl (meth)acrylates having the general composition: | | | | | |
| R_{F} - A - [CH₂]_{c}CR₂R₃OCOCH=CH₂ (method D) | | | | | |
| or | | | | | |
| R_{F} - A - [CH₂]_{c}CR₂R₃OCOCCH₃=CH₂ (method E) | | | | | |

| Ex. No. (Meth)acrylate | Ex. No. Alcohol | c | R₂ | R₃ | Method |
|---|---|---|---|---|---|
| 33 | 22 | 2 | -H | -H | D |
| 34 | 23 | 10 | -H | -H | D |
| 35 | 24 | 1 | -H | -H | D |
| 36 | 25 | 1 | -H | -CH₃ | D |
| 37 | 26 | 2 | -H | -H | D |
| 38 | 28 | 1 | -H | -H | D |
| 39 | 29 | 1 | -H | -H | E |
| 40 | 30 | 3 | -H | -H | E |

### EXAMPLE 41:

### Preparation of a dispersion for textile finishing (recipe 1)

The dispersion was prepared by intensively stirring the following components in a four neck flask equipped with stirrer, reflux condenser, inert gas supply and internal thermometer:
- 37.5 g: of polyfluoroalkyl acrylate (from Example 31)
- 31.0 g: of stearyl acrylate (SAC)
- 5.0 g: of glycidyl methacrylate (GMA)
- 4.5 g: of hydroxyethyl methacrylate (HEMA)
- 30.0 g: of dipropylene glycol
- 0.4 g: of dodecanethiol
- 6.0 g: of lauryl alcohol/16 ethylene oxide adduct (nonionic surfactant A)
- 4.5 g: of N,N-dimethyldodecylammonium acetate (cationic surfactant A)
- 200.0 g: of water

The emulsion was heated to 60°C under a constant stream of nitrogen. Then, 0.2 g of the initiator 2,2'-azo-bis-isobutyronitrile (AIBN) was added. The polymerization time was 10 hours at 60°C.

The resulting dispersion had a solids content of about 34%. For finishing textiles, the dispersion was acidified and diluted to 30 g/l. The dispersion was applied to fibrous substrates on an HVF 59301 laboratory pad-mangle from Mathis AG (Switzerland) followed by drying and heat treatment at 160°C/30 seconds in an LTE laboratory dryer from Mathis AG (Switzerland). The commercially available textile Sahara 530306 from NEL GmbH, Neugersdorf, was used as PES/Co 65/35 substrate to compare the applications. The wet pick-up was about 66% for all examples recited. The washing/drying procedure included 5 wash cycles at 60°C. The corresponding pieces of fabric were made up with ballast fabric to a wash load of one kilogram. The amount of laundry detergent needed was 7 g of "Coral intensive" per wash cycle. The fabric pieces were not dried between the wash cycles. After washing, the laundry was dried in a laundry dryer.

### EXAMPLE 42:

### Preparation of a dispersion for textile finishing (recipe 2)

To prepare the dispersion, the following components were intensively stirred under an inert gas atmosphere in an autoclave equipped with a stirrer, reflux condenser and internal thermometer:

| | |
|---|---|
| 69.5 g | of polyfluoroalkyl acrylate (from Example 31) |
| 19.0 g | of lauryl acrylate (LA) |
| 8.5 g | of vinyl chloride (VC) |
| 2.5 g | of N-methoxymethylacrylamide (N-MAM) |
| 3.5 g | of hydroxyethyl methacrylate |
| 30.0 g | of dipropylene glycol |
| 0.5 g | of dodecanethiol |
| 7.0 g | of stearyl/11 ethylene oxide adduct (nonionic surfactant B) |
| 4.0 g | of lauryltrimethylammonium chloride (cationic surfactant B) |
| 200.0 g | of water |

After the emulsion had been heated to 60°C, 0.6 g of the initiator 2,2'-azo-bis-2-amidinopropane dihydrochloride was added. The polymerization time was 6 hours at 60°C. After the reaction, the excess of vinyl chloride was stripped off.

The resulting dispersion had a solids content of about 38%. For finishing of textiles, the dispersion was acidified and diluted to 30 g/l. Application to textile substrates was carried as described in Example 41.

### EXAMPLE 43:

### Preparation of a dispersion for textile finishing (recipe 3)

To prepare the dispersion, the following components were intensively stirred under an inert gas atmosphere in an autoclave equipped with a stirrer, reflux condenser and internal thermometer:

| | |
|---|---|
| 60.5 g | of polyfluoroalkyl acrylate (from Example 31) |
| 12.5 g | of 2-ethylhexyl acrylate (2-EHAC) |
| 15.0 g | of vinylidene chloride (VDC) |
| 3.5 g | of N-methoxymethylacrylamide |
| 1.0 g | of hydroxyethyl methacrylate |
| 35.0 g | of dipropylene glycol |
| 0.7 g | of dodecanethiol |
| 6.0 g | of stearyl/11 ethylene oxide adduct (nonionic surfactant B) |
| 5.0 g | of sodium dodecylsulphate (SDS) |
| 200.0 g | of water |

After the emulsion had been heated to 60°C, 0.5 g of the initiator 2,2'-azo-bis-2-amidinopropane dihydrochloride was added. The polymerization time was 6 hours at 60°C. After the reaction, the excess of vinylidene chloride was stripped off.

The resulting dispersion had a solids content of about 36%. The dispersion was acidified and admixed with Cassurit HML (Clariant) and 20% by weight aqueous magnesium chloride solution, so that the concentration per 1 of liquor was in each case 30 g. Application to textile substrates was carried out as described in Example 41.

The results of isopropanol repellency (IPA), oil repellency (oleo) and water repellency (hydro) are reported in Table 7.

### EXAMPLES 44-47:

### Preparation, application and testing of dispersions for textile finishing similarly to Example 41

The formulation of the dispersions and also the results of isopropanol repellency (IPA), oil repellency (oleo) and water repellency (hydro) are reported in Table 7.

### EXAMPLES 48-51:

### Preparation, application and testing of dispersions for textile finishing similarly to Example 42

The formulation of the dispersions and also the results of isopropanol repellency (IPA), oil repellency (oleo) and water repellency (hydro) are reported in Table 7.

### EXAMPLES 52-55:

### Preparation, application and testing of dispersions for textile finishing similarly to Example 43

The formulation of the dispersions and also the results of isopropanol repellency (IPA), oil repellency (oleo) and water repellency (hydro) are reported in Table 7.

**TABLE 7 Preparation, application and testing of dispersions for textile finishing**

| | | Examples | | | | | | | | | | | | | | |
|---|---|---|---|---|---|---|---|---|---|---|---|---|---|---|---|---|
| | | Recipe 1 | | | | | Recipe 2 | | | | | Recipe 3 | | | | |
| | | 41 | 44 | 45 | 46 | 47 | 42 | 48 | 49 | 50 | 51 | 43 | 52 | 53 | 54 | 55 |
| Polyfluoroacrylate No. | | 31 | 35 | 37 | 38 | 39 | 31 | 32 | 33 | 34 | 37 | 31 | 32 | 36 | 39 | 40 |
| Polyfluoroacrylate amount | | 37.5 | 37.5 | 37.5 | 37.5 | 37.5 | 69.5 | 69.5 | 69.5 | 69.5 | 69.5 | 60.5 | 60.5 | 60.5 | 60.5 | 60.5 |
| SAC | | 31.0 | 31.0 | 31.0 | 31.0 | 31.0 | - | - | - | - | - | - | - | - | - | - |
| LA | | - | - | - | - | - | 19 | 19 | 19 | 19 | 19 | - | - | - | - | - |
| 2-EHAC | | - | - | - | - | - | - | - | - | - | - | 12.5 | 12.5 | 12.5 | 12.5 | 12.5 |
| VC | | - | - | - | - | - | 8.5 | 8.5 | 8.5 | 8.5 | 8.5 | - | - | - | - | - |
| VDC | | - | - | - | - | - | - | - | - | - | - | 15 | 15 | 15 | 15 | 15 |
| GMA | | 5.0 | 5.0 | 5.0 | 5.0 | 5.0 | - | - | - | - | - | - | - | - | - | - |
| N-MAM | | - | - | - | - | - | 2.5 | 2.5 | 2.5 | 2.5 | 2.5 | 3.5 | 3.5 | 3.5 | 3.5 | 3.5 |
| HEMA | | 4.5 | 4.5 | 4.5 | 4.5 | 4.5 | 3.5 | 3.5 | 3.5 | 3.5 | 3.5 | 1.0 | 1.0 | 1.0 | 1.0 | 1.0 |
| Nonionic surfactant A | | 6.0 | 6.0 | 6.0 | 6.0 | 6.0 | - | - | - | - | - | - | - | - | - | - |
| Nonionic surfactant B | | - | - | - | - | - | 7.0 | 7.0 | 7.0 | 7.0 | 7.0 | 6.0 | 6.0 | 6.0 | 6.0 | 6.0 |
| Cationic surfactant A | | 4.5 | 4.5 | 4.5 | 4.5 | 4.5 | - | - | - | - | - | - | - | - | - | - |
| Cationic surfactant B | | - | - | - | - | - | 4.0 | 4.0 | 4.0 | 4.0 | 4.0 | - | - | - | - | - |
| SDS | | - | - | - | - | - | - | - | - | - | - | 5.0 | 5.0 | 5.0 | 5.0 | 5.0 |
| Dipropylene glycol | | 30.0 | 30.0 | 30.0 | 30.0 | 30.0 | 30.0 | 30.0 | 30.0 | 30.0 | 30.0 | 35.0 | 35.0 | 35.0 | 35.0 | 35.0 |
| Prior to washing | IPA | 90 | 60 | 80 | 80 | 100 | 95 | 100 | 90 | 80 | 90 | 90 | 95 | 60 | 90 | 60 |
| | Oleo | 5-6 | 5 | 5 | 6 | 6-7 | 6 | 4-5 | 5 | 4 | 6 | 5-6 | 5 | 4-5 | 6-7 | 4-5 |
| | Hydro | 100 | 90 | 100 | 100 | 100 | 100 | 80 | 100 | 80 | 100 | 100 | 90-100 | 80-90 | 100 | 80 |
| After 5 washes | IPA | 50 | 40-50 | 60 | 80 | 90 | 90 | 90 | 80 | 80 | 80 | 90 | 90 | 50 | 80 | 40-50 |
| | Oleo | 4-5 | 4-5 | 5 | 6 | 6 | 5-6 | 4-5 | 5 | 3-4 | 5-6 | 5 | 5 | 4 | 6 | 4-5 |
| | Hydro | 100 | 90 | 100 | 100 | 90-100 | 100 | 70-80 | 100 | 80 | 100 | 100 | 80 | 80 | 90-100 | 80 |

## Claims

1. Fluorous telomeric compounds of the formula IV:
R_{F} - A - [CH₂]_{c}CR₂R₃ - Z (IV)
where R_{F} is a perfluoroalkyl radical of 1 to 20 carbon atoms, A is a group of the formulae 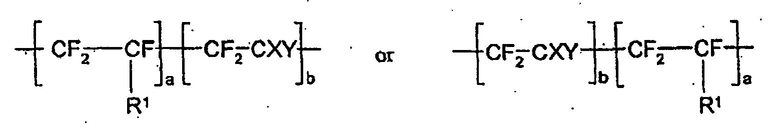 R¹ is CF₃ OR₄, Cl, Br or I,
R₂ and R₃ are H, alkyl or aryl
R₄ is perfluoromethyl, perfluoropropyl or perfluoropropyloxypropyl
X and Y are H, Cl or F
Z is -OH, -OCOCH=CH₂ or -OCOCCH₃=CH₂
a is from a>0 to 10, b is from 1 to 30 and c is from 1 to 30
**characterized in that**
the molecular weight of the compounds of the formula IV is more than 750 g/mol.

2. Compounds according to Claim 1, **characterized in that** R₁ is Cl.

3. Compounds according to Claim 1, **characterized in that** R₁ is CF₃.

4. Compounds according to Claim 1, **characterized in that** X and Y are F or X is F and Y is Cl or X and Y are hydrogen.

5. Compounds according to Claim 1, **characterized in that** a is from a>0 to 5

6. Compounds according to Claim 1, **characterized in that** c is 1; and R₂ and R₃ are H br CH₃.

7. Compounds according to Claim 1, **characterized in that** c is 2; and R₂ and R₃ are H or CH₃.

8. Compounds according to Claim 1, **characterized in that** R_{F} is a polyfluoroalkyl radical of 1 to 3 fluorinated carbon atoms.

9. Compounds according to Claim 1, **characterized in that** R_{F} is a polyfluoroalkyl radical of 4 to 16 fluorinated carbon atoms.

10. Compounds according to Claim 1, **characterized in that** a + b is > 3.

11. Copolymers containing a monomer of the formula IV where Z is -OCOCH=CH₂ or -OCOCCH₃=CH₂, one or more nonfluorous polymerizable vinyl monomers, one or more thermally crosslinkable or isocyanate-reactive monomers and optionally a chlorine-containing polymerizable vinyl monomer.

12. Copolymers containing; based on the total weight of the copolymer:
a) 20% to 97% by weight and preferably 40% to 90% by weight of a monomer of the formula IV where Z is -OCOCH=CH or -OCOCCH₃=CH,
b) 10% to 50% by weight of one or more nonfluorous polymerizable vinyl monomers and/or
c) 0.5% to 20% by weight and preferably 1% to 10% by weight of one or more thermally crosslinkable or isocyanate-reactive monomers.

13. Copolymers containing, based on the total weight of the copolymer:
a) 40% to 90% by weight and preferably 45% to 85% by weight of a monomer of the formula IV where Z is -OCOCH=CH or -OCOCCH₃=CH,
b) 0% to 50% by weight and preferably 0.01% to 30% by weight of one or more nonfluorous polymerizable vinyl monomers and/or
c) 0.5% to 20% by weight and preferably 1% to 10% by weight of one or more thermally crosslinkable or isocyanate-reactive monomers and
d) 0.5% to 50% by weight and preferably 2% to 30% by weight of a chlorine-containing polymerizable vinyl monomer.

14. Use of the copolymers according to Claims 11 to 13 for water-, oil- and soil-repellent finishing of fibrous substrates.

## Patentansprüche

1. Fluorhaltige Telomerverbindungen der Formel IV:
R_{F} - A - [CH₂]_{c}CR₂R₃ - Z (IV)
worin R_{F} ein Perfluoralkylrest mit 1 bis 20 C-Atomen ist, A eine Gruppe der Formeln 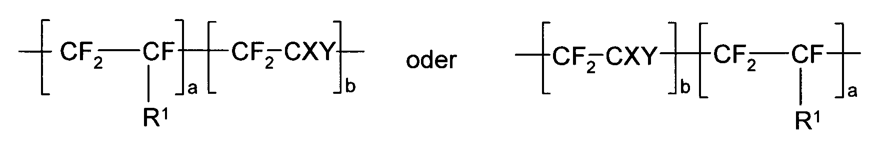 R¹ CF₃ OR₄, Cl, Br oder I,
R₂ und R₃ H, Alkyl oder Aryl
R₄ Perfluormethyl, Perfluorpropyl oder Perfluorpropyloxypropyl
X und Y H, Cl oder F
Z -OH, -OCOCH = CH₂ oder -OCOCCH₃ = CH₂
a eine Zahl von a>0 bis 10, b eine Zahl von 1 bis 30 und c eine Zahl von 1 bis 30 bedeutet, **dadurch gekennzeichnet, dass** das Molekulargewicht der Verbindungen der Formel IV mehr als 750 g/Mol beträgt.

2. Verbindungen gemäß Anspruch 1, **dadurch gekennzeichnet, dass** R₁ = Cl bedeutet.

3. Verbindungen gemäß Anspruch 1, **dadurch gekennzeichnet, dass** R₁ = CF₃ bedeutet.

4. Verbindungen gemäß Anspruch 1, **dadurch gekennzeichnet, dass** X und Y F oder X F und Y Cl oder X und Y Wasserstoff bedeuten.

5. Verbindungen gemäß Anspruch 1, **dadurch gekennzeichnet, dass** a eine Zahl von a>0 bis 5 ist.

6. Verbindungen gemäß Anspruche 1, **dadurch gekennzeichnet, dass** c 1; und R₂ und R₃ H oder CH₃ sind.

7. Verbindungen gemäß Anspruch 1, **dadurch gekennzeichnet, dass** c 2; und R₂ und R₃ H oder CH₃ sind.

8. Verbindungen gemäß Anspruch 1, **dadurch gekennzeichnet, dass** R_{F} ein Polyfluoralkylrest mit 1 bis 3 fluorierten Kohlenstoffatomen darstellt.

9. Verbindungen gemäß Anspruch 1, **dadurch gekennzeichnet, dass** R_{F} ein Polyfluoralkylrest mit 4 bis 16 fluorierten Kohlenstoffatomen darstellt.

10. Verbindungen gemäß Anspruch 1, **dadurch gekennzeichnet, dass** a + b > 3 ist.

11. Copolymere enthaltend ein Monomer der Formel IV wobei Z -OCOCH=CH₂ oder-OCOCCH₃=CH₂ bedeutet, eines oder mehrere nicht-fluorhaltige polymerisierbare Vinylmonomeren, eines oder mehrere thermisch vernetzbare oder Isocyanat-reaktive Monomeren und gegebenenfalls ein chlorhaltiges polymerisierbares Vinylmonomer.

12. Copolymere enthaltend, bezogen auf das Gesamtgewicht des Copolymeren:
a) 20 bis 97 Gew.-%, vorzugsweise 40 bis 90 Gew.-% eines Monomeren der Formel IV, wobei Z -OCOCH=CH oder-OCOCCH₃=CH bedeutet,
b) 10 bis 50 Gew.-% eines oder mehrerer nicht-fluorhaltiger polymerisierbarer Vinyl-Monomeren und/oder
c) 0,5 bis 20 Gew.-%, vorzugsweise 1 bis 10 Gew.-% eines oder mehrerer thermisch vernetzbarer oder Isocyanat-reaktiver Monomeren.

13. Copolymere enthaltend, bezogen auf das Gesamtgewicht des Copolymeren:
a) 40 bis 90 Gew.-%, vorzugsweise 45 bis 85 Gew.-% eines Monomeren der Formel IV, wobei Z -OCOCH=CH oder-OCOCCH₃=CH bedeutet,
b) 0 bis 50 Gew.-%, vorzugsweise 0,01 bis 30 Gew.-% eines oder mehrerer nicht-fluorhaltiger polymerisierbarer Vinyl-Monomeren und/oder
c) 0,5 bis 20 Gew.-%, vorzugsweise 1 bis 10 Gew.-% eines oder mehrerer thermisch vernetzbarer oder Isocyanat-reaktiver Monomeren und
d) 0,5 bis 50 Gew.-%, vorzugsweise 2 bis 30 Gew.-% eines chlorhaltigen polymerisierbaren Vinyl-Monomeren.

14. Verwendung der Copolymeren gemäß Ansprüchen 11 bis 13 zur wasser-, öl- und schmutzabweisenden Ausrüstung von faserigen Substraten.

## Revendications

1. Composés télomères fluorés de formule IV :
R_{F} - A - [CH₂]_{c}CR₂R₃ - Z (IV)
dans laquelle R_{F} est un radical perfluoroalkyle de 1 à 20 atomes de carbone,
A est un groupe de formule 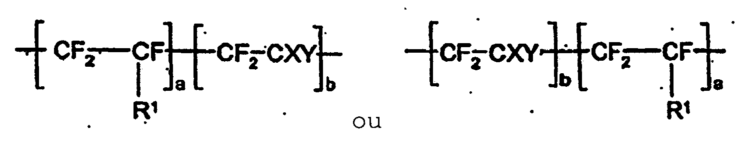 R¹ est CF₃, OR₄, Cl, Br ou I,
R₂ et R₃ sont H, alkyle ou aryle,
R₄ est perfluorométhyle, perfluoropropyle ou perfluoropropyloxypropyle,
X et Y sont H, Cl ou F,
Z est -OH, -OCOCH=CH₂ ou -OCOCCH₃=CH₂,
a est de a > 0 à 10, b est de 1 à 30 et c est de 1 à 30,
**caractérisés en ce que**
le poids moléculaire des composés de formule IV est supérieur à 750 g/mol.

2. Composés selon la revendication 1, **caractérisés en ce que** R₁ est Cl.

3. Composés selon la revendication 1, **caractérisés en ce que** R₁ est CF₃.

4. Composés selon la revendication 1, **caractérisés en ce que** X et Y sont F ou X est F et Y est C1 ou X et Y sont l'hydrogène.

5. Composés selon la revendication 1, **caractérisés en ce que** a est de a > 0 à 5.

6. Composés selon la revendication 1, **caractérisés en ce que** c est 1 ; et R₂ et R₃ sont H ou CH₃.

7. Composés selon la revendication 1, **caractérisés en ce que** c est 2 ; et R₂ et R₃ sont H ou CH₃.

8. Composés selon la revendication 1, **caractérisés en ce que** R_{F} est un radical polyfluoroalkyle de 1 à 3 atomes de carbone fluorés.

9. Composés selon la revendication 1, **caractérisés en ce que** R_{F} est un radical polyfluoroalkyle de 4 à 16 atomes de carbone fluorés.

10. Composés selon la revendication 1, **caractérisés en ce que** a + b est > 3.

11. Copolymères contenant un monomère de formule IV, dans laquelle Z est -OCOCH=CH₂ ou -OCOCCH₃=CH₂, un ou plusieurs monomères vinyliques polymérisables non fluorés, un ou plusieurs monomères réticulables thermiquement ou réactifs avec les isocyanates et éventuellement un monomère vinylique polymérisable contenant du chlore.

12. Copolymères contenant, par rapport au poids total du copolymère :
a) 20 % à 97 % en poids et de préférence 40 % à 90 % en poids d'un monomère de formule IV dans laquelle Z est - OCOCH=CH ou -OCOCCH₃=CH,
b) 10 % à 50 % en poids d'un ou de plusieurs monomères vinyliques polymérisables non fluorés et/ou
c) 0,5 % à 20 % en poids et de préférence 1 % à 10 % en poids d'un ou de plusieurs monomères réticulables thermiquement ou réactifs avec les isocyanates.

13. Copolymères contenant, par rapport au poids total du copolymère :
a) 40 % à 90 % en poids et de préférence 45 % à 85 % en poids d'un monomère de formule IV dans laquelle Z est - OCOCH=CH ou -OCOCCH₃=CH,
b) 0 % à 50 % en poids et de préférence 0,01 % à 30 % en poids d'un ou de plusieurs monomères vinyliques polymérisables non fluorés et/ou
c) 0,5 % à 20 % en poids et de préférence 1 % à 10 % en poids d'un ou de plusieurs monomères réticulables thermiquement ou réactifs avec les isocyanates et
d) 0,5 % à 50 % en poids et de préférence 2 % à 30 % en poids d'un monomère vinylique polymérisable contenant du chlore.

14. Utilisation des copolymères selon les revendications 11 à 13 pour la finition anti-eau, anti-huile et anti-salissure de substrats fibreux.
